# EUROPEAN PATENT APPLICATION

(11) **EP 3 435 081 A1**
(43) Date of publication of application: **30.01.2019**
(21) Application number: 16895377.6
(22) Date of filing: 23.03.2016
(51) Int. Cl.: G01N 33/543

(54) **METHOD FOR FORMING SAMPLE ADDITION PART OF IMMUNOCHROMATOGRAPHIC TEST DEVICE AND IMMUNOCHROMATOGRAPHIC TEST DEVICE**

(71) Applicant: Denka Seiken Co., Ltd., Chuo-ku Tokyo 103-8338 (JP)
(72) Inventor: SHINOHARA, Yuki, Gosen-shi Niigata 959-1834 (JP); KUWAHARA, Miwa, Gosen-shi Niigata 959-1834 (JP); AKITA, Hiroshi, Gosen-shi Niigata 959-1834 (JP); MIYAZAWA, Takashi, Gosen-shi Niigata 959-1834 (JP)
(74) Representative: Mewburn Ellis LLP
(86) International application number: PCT/JP2016/059218
(87) International publication number: WO 2017/163341

(57) **Abstract**

Disclosed are an immunochromatographic test device which allows a target substance to be detected or quantified more rapidly and sensitively than conventional methods, and a method of forming a sample addition part of the test device. The method of forming a sample addition part of the immunochromatographic test device includes applying a surfactant that is in white powder form under normal conditions, such as sodium deoxycholate, to the sample addition part and drying it. The immunochromatographic test device includes the sample addition part formed by the method.

## Description

### TECHNICAL FIELD

The present invention relates to a method of forming a sample addition part of an immunochromatographic test device and to an immunochromatographic test device having the sample addition part formed by the method.

### BACKGROUND ART

Recently, simple test reagents and kits for performing various tests in a short time, for example, for detecting infections by pathogens such as viruses and bacteria, as well as for detecting pregnancy have been developed. The targets of the detection or quantification are constituents of pathogens, human chorionic gonadotropin or the like. Many of the reagents have characteristics that no special equipment is required, the operation is easy and the price is inexpensive. For example, simple test reagents for pregnancy diagnosis are commercially available in general drugstores. Unlike other test reagents, simple test reagents for checking pathogen infections are widely used not only in large hospitals and medical examination centers, but also in general hospitals and clinics. Since these general hospitals and clinics are often medical institutions where patients first visit, on-the-spot determination of the presence of infections in samples obtained from the patients can allow early therapeutic treatment. Thus, simple test reagents are increasingly important in medical care.

As simple test methods, immunoassays utilizing antigen-antibody reaction, in particular immunochromatography assays, are known in general. In immunochromatography, a complex of a trapper (capturing substance) that binds specifically to a target substance and a labeled substance that binds specifically to the target substance is formed on a membrane, and the label is detected/quantified to detect (measure or quantify) the target substance. Since immunochromatography can be carried out with a simple measurement device and is also excellent in cost, it is widely used to detect various target substances.

In one example, immunochromatography comprises adding a test sample containing a target substance dropwise to a test device comprising a detection part formed by immobilizing an antibody that binds specifically to the target substance on a membrane strip such as nitrocellulose as a capturing substance, and a labeled substance part containing a labeled substance that binds specifically to the target substance; developing the test sample along the strip while forming a complex of the target substance and the labeled substance; and detecting or quantifying the label by capturing the complex in the detection part (see, Patent Documents 1 and 2).

Recently, techniques wherein a test sample is pretreated on a sample addition part of an immunochromatographic test device by pre-impregnating the sample addition part with a reagent have been developed.

### PRIOR ART REFERENCES

### PATENT DOCUMENTS

Patent Document 1: JP 2008-268043 A
Patent Document 2: JP 2008-203135 A

### SUMMARY OF THE INVENTION

### PROBLEMS TO BE SOLVED BY THE INVENTION

However, the concentration of the reagent impregnated in the sample addition part can vary due to, for example, uneven drying. As a result, when a test sample is added to the sample addition part, the reagent impregnated in the sample addition part and the test sample may not be mixed well, and thus the test sample may not be developed smoothly on the membrane. The fact that the test sample is not developed rapidly on the membrane has led to problems of slowed measurement speed and increased background due to retention of the test sample on the membrane.

Accordingly, objects of the present invention are to remedy the above-mentioned problems and provide an immunochromatographic test device which allows a target substance to be detected or quantified more rapidly and sensitively than conventional methods, and a method of forming a sample addition part of the test device.

### MEANS FOR SOLVING THE PROBLEMS

As a result of intensive studies, the present inventors have found that, in immunochromatography using a porous support such as nitrocellulose membrane, use of a test device comprising a sample addition part which has an improved recoverability of the reagents impregnated therein enables rapid and highly sensitive measurement, thereby completing the present invention.

Accordingly, the present invention provides the following (1) to (5).
(1) A method of forming a sample addition part of an immunochromatographic test device, the method comprising applying a surfactant(s) that is/are in white powder form under normal conditions to a sample addition part, and drying the sample addition part.
(2) The method according to item (1), wherein the surfactant is an anionic, nonionic or amphoteric surfactant having a molecular weight of from 200 to 900.
(3) The method according to item (1), wherein the surfactant is at least one selected from the group consisting of deoxycholic acid and salts thereof, and cholic acid and salts thereof.
(4) The method according to item (3), wherein the surfactant is sodium deoxycholate.
(5) An immunochromatographic test device comprising a sample addition part formed by the method according to any one of items (1) to (4).

### EFFECTS OF THE INVENTION

The use of a test device comprising the sample addition part formed by using the method of the present invention provides well mixing of a test sample and a reagent impregnated in the sample addition part, which allows the test sample to rapidly develop on a membrane, thus enabling a low background, low false-positive, fast and high-sensitive measurement.

### BRIEF DESCRIPTION OF THE DRAWINGS

Fig. 1 shows top and cross-sectional views of the test device according to one embodiment of the present invention.
Fig. 2 shows the difference in the development of a red dye dependent on the presence of a surfactant on the sample addition part, observed in Examples and Comparative Examples below.
Fig. 3 shows the difference in color remaining of a red dye on the sample addition part dependent on the presence of a surfactant on the sample addition part, observed in Examples and Comparative Examples below.
Fig. 4 shows the difference in reproducibility of immunoassay results dependent on the presence of a surfactant on the sample addition part, observed in Examples and Comparative Examples below.

### DETAILED DESCRIPTION OF THE INVENTION

As used herein, the term "sample addition part" refers to a site in an immunochromatographic test device to which a test sample is added. The sample addition part is formed by placing a pad comprising a porous support with water absorbing property, for example, sponge or nonwoven fabrics such as glass fiber, on a porous support such as nitrocellulose membrane which constitutes the device body. When a pad is used, the pad may be referred to as sample addition pad. Alternatively, instead of forming the sample addition part into a pad, a partial region of the porous support such as nitrocellulose membrane which constitutes the device body can also be used as the sample addition part. Preferably, the sample addition part is arranged on one end of the test strip.

A reagent necessary for an antigen-antibody reaction can be previously applied to the sample addition part. For example, a surfactant may be applied so as to prevent involvement of nonspecific components contained in a test sample in the antigen-antibody reaction, or a hemolytic agent may be applied when assaying blood samples. In immunochromatography, a test sample is often suspended before it is added to the sample addition part. In that case, however, dilution of the test sample also results in dilution of the target substance, which will impair the detection sensitivity. Therefore, buffer agents, surfactants, various proteins, salts, and sugars, which are the ingredients of the test sample suspension, may be pre-applied to the sample addition part.

The method of the present invention requires application of a surfactant that is in white powder form under normal conditions to a sample addition part. The present inventors intensively studied to find that this application enables rapid development of a test sample on the membrane and high-sensitive detection of a target substance in immunochromatography, thereby completing the present invention. As used herein, a surfactant which is "in white powder form under normal conditions" means that the surfactant is in white powder form when it exists alone at normal temperature (25°C) under normal pressure (1 atm). Other than the surfactant, use of liquid, pasty, or waxy surfactants may result in insufficient drying, problem of stability of sample addition part, and in insufficiency of rapid penetration effect when a sample is added, and thus is not suitable for the present invention. Since the surfactant is generally dissolved in an aqueous medium and applied to a porous support, surfactants excellent in water solubility are preferred. Among surfactants in white powder form, surfactants having a molecular weight of from 200 to 900 are preferred because of their excellent water solubility and stability, so that the effect of the present invention can be obtained.

Examples of such a surfactant that is in white powder form under normal conditions and excellent in water solubility include surfactants below. These surfactants may be used individually or two or more of them may be used in combination.
sodium cholate,
sodium deoxycholate,
sodium lauryl sulfate,
sucrose monocholate,
β-D-fructopyranosyl-α-D-glucopyranoside monododecanoate,
β-D-fructopyranosyl-α-D-glucopyranoside monodecanoate,
n-octanoyl-*N*-methylglucamide,
n-nonanoyl-*N*-methylglucamide,
n-decanoyl-*N*-methylglucamide,
n-octyl-β-D-thioglucopyranoside,
n-octyl-β-D-maltopyranoside,
n-octyl-β-D-glucopyranoside,
n-nonyl-β-D-thiomaltopyranoside,
n-dodecyl-β-D-maltopyranoside,
n-decyl-β-D-maltopyranoside,
*N*,*N*-bis(3-D-gluconamidopropyl)cholamide,
*N*,*N*-bis(3-D-gluconamidopropyl)deoxycholamide,
3-[(3-cholamidopropyl)dimethylammonio]propanesulfonic acid,
3-[(3-cholamidopropyl)dimethyl ammonio]-2-hydroxypropanesulfonic acid, ZWITTERGENT 3-10 Detergent (trademark) manufactured by Calbiochem, ZWITTERGENT 3-12 Detergent (trademark) manufactured by Calbiochem, and ZWITTERGENT 3-14 Detergent (trademark) manufactured by Calbiochem. Among these surfactants, at least one which is anionic and selected from the group consisting of deoxycholic acid and salts thereof, and cholic acid and salts thereof is preferred. In particular, sodium cholate and sodium deoxycholate are more preferred because of their excellent water solubility and inexpensiveness.

Preferred methods for applying the above surfactant to the sample addition part include dissolving the surfactant into a solution, preferably an aqueous solution containing water or an aqueous buffer as a solvent, and adding the aqueous solution to the sample addition part by impregnation or dropping. In this case, the concentration of the surfactant in the surfactant solution is usually from 0.005% by mass to 5% by mass, preferably from 0.05% by mass to 2% by mass. The amount of the surfactant added to the sample addition part is usually about from 0.0013 mg to 10 mg, preferably about from 0.005 mg to 4 mg.

The effect of applying the surfactant is thought to be obtained as follows: when the above-described suspension is added to the porous support and dried, the surfactant exists in the form of fine crystals in accordance with evaporation of water molecules on the sample addition part, and this surfactant helps the test sample to penetrate evenly into the sample addition part and thereby to develop rapidly on the membrane. In addition, when a reagent necessary for an antigen-antibody reaction is pre-applied to the sample addition part, uneven concentration and self-aggregation during drying of the reagent necessary for the antigen-antibody reaction can be remarkably prevented. The prevention of uneven concentration allows the test sample and the reagent necessary for the antigen-antibody reaction to be mixed evenly and allows rapid development on the membrane. Furthermore, the prevention of self-aggregation can dramatically increase the signal to noise ratio without background or false positive due to self-aggregation. Since the surfactant is in white powder form under normal conditions, it can be kept in a dry state, has good storage stability and is easy to handle. In addition, since the surfactant is white, it does not interfere with the visual judgment of test results.

The target substance or test sample to be detected using the immunochromatographic test device of the present invention is not restricted. Examples of the test sample include pharyngeal or nasal swab, nasal aspirate, pharyngeal or nasal lavage, saliva, serum, plasma, whole blood, stool suspension, urine, culture medium and dilutions thereof with buffers. The test samples may also be used directly without dilution. The target substance is not restricted and may be any substance to be detected. Specific examples of the target substance include viral antigens such as influenza virus, adenovirus, RS virus, HAV, HBs, HIV, and norovirus; bacterial antigens such as MRSA, group A hemolytic streptococcus, group B hemolytic streptococcus, and Legionella sp.; and antigens such as toxins produced by bacteria or the like, Mycoplasma, Chlamydia trachomatis, hormones such as human chorionic gonadotropin, C-reactive protein, myoglobin, cardiac troponin, various tumor markers, agricultural chemicals, and environmental hormones, as well as antibodies against the above bacteria and viruses.

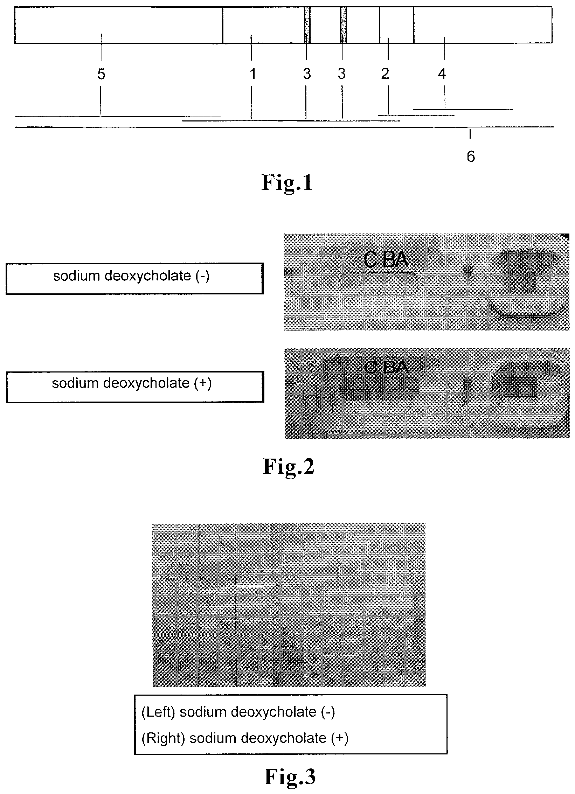

The present invention also provides an immunochromatographic test device comprising a sample addition part formed as described above. The constitution *per se* of the test device may be the same as those of well-known ones described in, for example, Patent Documents 1 and 2, except that the surfactant that is in white powder form under normal conditions is applied to the sample addition part. A specific embodiment of the immunochromatographic test device of the present invention is schematically shown in Fig. 1, but the test device of the present invention is not limited thereto.

In Fig. 1, the upper drawing shows a top view, and the lower drawing shows a cross-sectional view. In the specific embodiment shown, a nitrocellulose membrane (1) with two detection parts (3) formed thereon; an absorption pad part (5) made of filter paper; a labeled substance part (2) formed according to the above method; and a sample addition part (4) made of glass fiber filter are laminated on a plastic plate (6). Further, as illustrated, one end region of the absorption pad part (5) is laid on one end region of the nitrocellulose membrane (1), the other end region of the nitrocellulose membrane (1) is laid on one end region of the labeled substance part (2), and the other end region of the labeled substance part (2) is laid on one end region of the sample addition part (4), whereby a continuous lateral flow path is formed.

Next, the immunoassay using the test device will be explained. First, a test sample is prepared by suspension/extraction of a sample in a sample suspension/extraction buffer. The test sample is added dropwise to the sample addition part (4) of the test device, wherein the test device comprises, on a nitrocellulose membrane (1) laminated on a plastic plate (6), the labeled substance part (2) comprising a stabilized dried labeled substance pad obtained by labeling an antibody which undergoes an antigen-antibody reaction with a target substance with a colored latex particle, according to the above-mentioned method; and the detection parts (3) obtained by immobilizing, as a capturing substance, an antibody which undergoes an antigen-antibody reaction with the target substance, in a line shape. The test sample containing the target substance moves horizontally on the membrane while developing the labeled substance. Thus, in the presence of the target substance, a first complex of the target substance and the labeled substance is formed. Further, when the first complex reaches the detection part (3), a second complex of the capturing antibody-the target substance-the labeled substance is formed on the line. By virtue of the colored latex particle contained in the second complex, the existence of the second complex is detected and, in turn, the presence or absence of the target substance in the sample is judged. Here, the detection part (3) is a region formed by immobilizing in a line shape an antibody or an antigen-binding fragment thereof which undergoes an antigen-antibody reaction with the target substance, which can bind to the target substance simultaneously with the binding of the antibody or the antigen-binding fragment thereof bound to the colored latex particle. Other components which did not participate in the reaction are absorbed in the absorption pad part (5). In the embodiment shown in Fig. 1, two detection parts (3) exist, in order to capture two types of target substances separately, such as influenza A virus and influenza B virus as described in the Example below. A plurality of detection parts (3) provided enable simultaneous immunoassays of a plurality of target substances.

The use of the test device of the present invention comprising a latex particle bound to an antibody or an antigen-binding fragment thereof which undergoes an antigen-antibody reaction with the target substance as a stabilized dried labeled substance enables rapid and simple measurement of the target substance.

### EXAMPLES

The present invention will now be described more specifically with reference to the following examples and comparative examples. However, the present invention is not limited to these Examples.

### Detection of influenza virus by immunochromatography

### 1. Preparation of anti-influenza virus monoclonal antibody

### (1) Anti-influenza A virus NP antibody

BALB/c mice were immunized with influenza A virus antigen and kept for a certain period of time. Then the spleen was removed from each mouse and the cells were fused with mouse myeloma cells (P3X63) according to the method by Kohler *et al* (Kohler et al., Nature, vol. 256, p495-497 (1975)). The obtained fused cells (hybridomas) were maintained at 37°C in an incubator and purified (into monoclonal cells) while checking the antibody activity in the supernatant by ELISA using a plate on which an influenza A virus NP antigen was immobilized. The obtained two cell lines were intraperitoneally administered into pristan-treated BALB/c mice, respectively. After about two weeks, ascites containing antibodies was collected. IgG antibodies in the collected ascites were purified by affinity chromatography on a protein A column to obtain two types of purified anti-influenza A virus NP antibodies.

### (2) Anti-influenza B virus NP antibody

Two types of purified anti-influenza B virus NP antibodies were obtained in the same manner as in (1) except that influenza B virus antigen was used.

### 2. Preparation of labeled substance pad

One type each of the purified anti-influenza A virus NP antibodies and of the purified anti-influenza B virus NP antibodies was used. The anti-influenza A virus antibody was covalently bound to a blue latex particle, and the conjugate was dispersed in a suspension and sonicated to prepare a fully dispersed suspension of anti-A type latex suspension. Similarly, an anti-B type latex suspension wherein an anti-influenza B antibody was covalently bound, was prepared . The anti-A type latex suspension and the anti-B type latex suspension were mixed and applied to a glass fiber having a size of 20 cm × 1 cm. Then the glass fiber was dried well under hot air to prepare a labeled substance pad on which the dried mixture was formed.

### 3. Preparation of sample addition pad

A glass fiber having a size of 2.0 cm × 20 cm was used.

### 4. Preparation of test device

A test device having the same configuration as shown in Fig. 1 was used. A nitrocellulose membrane was cut into a size of 2 cm × 20 cm and backed with a plastic plate with an adhesive. At positions of 0.6 cm and 1.0 cm from the bottom end of the nitrocellulose membrane, solutions of anti-influenza A virus antibody (different from the above one) and anti-influenza B virus antibody (different from the above one) in an amount for forming a line with a width of 1 mm were applied respectively to a length of 20 cm. The resultant was dried well under hot air to immobilize the antibodies (detection part). Next, a filter paper having a size of 3 cm × 20 cm was overlapped with the top end of the nitrocellulose membrane by 5 mm to provide an absorption pad part. Further, the labeled substance pad was overlapped with the bottom end of the nitrocellulose membrane by 2 mm to provide a labeled substance part. Still further, the sample addition pad was placed at a position 7 mm away from the top end of the labeled substance pad to provide a sample addition part. Then, the resultant was cut into strips with a width of 5 mm using a cutter to prepare an integrated test device.

### 5. Testing

### Example 1 and Comparative Example 1

First, the sample addition part of the test device prepared as above was impregnated with a solution containing sample developing ingredients (100 mM Tris-HCl (pH8.0), 1% Tween 20, 0.5% BSA) and an edible red dye (at a concentration of 0.1%) (Comparative Example 1). Alternatively, the sample addition part was impregnated with a solution further containing 0.2% by mass of sodium deoxycholate (DOC) in addition to the above ingredients (Example 1). A physiological saline as a sample was added dropwise to the sample addition part, and the degree of release of the edible dye immediately after dropping the sample and the appearance of the sample addition part after completion of the test were observed. The test device having the sample addition part containing sodium deoxycholate resulted in clearly higher amount of dye released immediately after the test (Fig. 2). Also, the sodium deoxycholate-containing sample resulted in less color remaining of the dye on the sample addition part after completion of the test (Fig. 3). From the above, it can be assumed that sodium deoxycholate contained in the sample addition part improved the release of the sample-developing ingredients, thereby also improving the release of the edible dye. Although the difference may be difficult to recognize in the black-and-white drawing, in a color photograph, the difference between Example 1 and Comparative Example 1 in Figs. 2 and 3 can be clearly recognized with naked eye.

### Example 2 and Comparative Example 2

Test devices having a sample addition part containing the above-described sample developing ingredients and sodium deoxycholate (Example 2) and having a sample addition part containing only the sample developing ingredients (Comparative Example 2) were prepared in the same manner as in Example 1 and Comparative Example 1, respectively, except that the solution with which the sample addition part was impregnated did not contain the red edible dye. Immunochromatography tests were carried out using an influenza virus B antigen on the test devices, and the variations in signal intensity were determined using an immunochromatographic reader (Hamamatsu Photonics K.K.) (each n = 10).

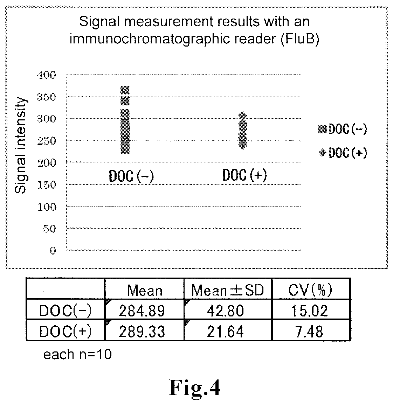

The results are shown in Fig. 4. As shown in Fig. 4, with the test device having the sample addition part containing sodium deoxycholate (Example 2), the variations in signal intensity were clearly smaller (see Fig. 4; sodium deoxycholate (+): CV = 7.48%, sodium deoxycholate (-): CV = 15.02%). It is seen from the above that use of the sample addition part containing sodium deoxycholate improved the release of the sample developing ingredients from the sample addition part, leading to the stable test results.

## Claims

1. A method of forming a sample addition part of an immunochromatographic test device, the method comprising:
applying a surfactant(s) to said sample addition part, wherein said surfactant(s) is/are in white powder form under normal conditions; and
drying said sample addition part.

2. The method according to claim 1, wherein said surfactant is an anionic, nonionic or amphoteric surfactant having a molecular weight of from 200 to 900.

3. The method according to claim 1, wherein said surfactant is at least one selected from the group consisting of deoxycholic acid and salts thereof, and cholic acid and salts thereof.

4. The method according to claim 3, wherein said surfactant is sodium deoxycholate.

5. An immunochromatographic test device, comprising said sample addition part formed by the method according to any one of claims 1 to 4.
